# EUROPÄISCHE PATENTSCHRIFT

(11) **EP 2 198 767 B1**
(45) Veröffentlichungstag und Bekanntmachung des Hinweises auf die Patenterteilung: **09.05.2018**
(21) Anmeldenummer: 09173118.2
(22) Anmeldetag: 15.10.2009
(51) Int. Cl.: A61B 1/00

(54) **Endoskop mit einem Endoskopschaft, in dem an seinem distalen Ende eine Abbildungsoptik angeordnet ist**
Endoscope with an endoscopic shaft comprising an imaging lens at its distal end
Endoscope doté d'un arbre d'endoscope dans lequel une optique de représentation est agencée à l'extrémité distale

(30) Priorität: 18.12.2008 DE 102008063619
(43) Veröffentlichungstag der Anmeldung: 23.06.2010
(73) Patentinhaber: Henke-Sass, Wolf GmbH, 78532 Tuttlingen (DE)
(72) Erfinder: Ivanovic, Boban, 78532, Tuttlingen (DE); Caruso, Nicolino, 78532, Tuttlingen (DE)
(74) Vertreter: Geyer, Werner

(56) Entgegenhaltungen:
- DE-A1- 10 205 735
- DE-A1- 10 225 378
- US-A- 4 805 598

## Beschreibung

Die vorliegende Erfindung betrifft ein Endoskop mit einem Endoskopschaft, der an seinem distalen Ende eine Beobachtungsöffnung aufweist, die mit einer transparenten Abdeckung hermetisch dicht verschlossen ist, und mit einer mehrere Optikelemente umfassenden Abbildungsoptik, die innerhalb des Endoskopschaftes im Bereich seines distalen Endes von der Abdeckung beabstandet angeordnet ist.

Bei einem solchen Endoskop, das beispielsweise aus der DE 42 11 547 A1 bekannt ist, besteht die Schwierigkeit, daß sich an der Innenseite der Abdeckung und am nähesten an der Abdeckung liegenden Optikelement der Abbildungsoptik Verschmutzungen ansammeln können, die die Abbildungsqualität deutlich verschlechtern. Solche Verschmutzungen können insbesondere durch das notwendige Autoklavieren auftreten, bei dem das Endoskop bzw. zumindest der Schaft jeweils mehrere Minuten in gesättigtem Wasserdampf bei bis zu 140°C sterilisiert wird. Diese Bedingungen können dazu führen, daß sich Verschmutzungen beispielsweise von dem Inneren des Endoskopschaftes lösen, ablagern und dadurch zu der unerwünschten Verschlechterung bei der Abbildung führen.

Die DE 102 25 378 A1 zeigt ein Endoskop mit einem Endoskopschaft, der an seinem distalen Ende eine Beobachtungsöffnung aufweist, die mit einer ersten Linse verschlossen ist. Daran schließt sich eine zweite Linse an, die mit der ersten Linse über ein Lötmittel so verbunden ist, dass der Zwischenraum zwischen beiden Linsen vollständig abgedichtet ist.

Die US 4,805,598 zeigt ein Endoskop mit einem Endoskopschaft, der an seinem distalen Ende eine Beobachtungsöffnung aufweist, die mit einer ersten Linse verschlossen ist. Von der ersten Linse beabstandet ist eine zweite Linse vorgesehen, wobei beide Linsen mit einem Linsenhalter verkittet sind. Der Zwischenraum zwischen beiden Linsen ist mit einer transparenten Substanz aufgefüllt.

Die DE 102 05 735 A1 beschreibt ein Endoskop mit einem Endoskopschaft, der an seinem distalen Ende eine Beobachtungsöffnung aufweist, die mit einer transparenten Abdeckung hermetisch dicht verschlossen ist. An der Innenseite der Abdeckung liegt ein O-Ring an, der einerseits einen Spalt zwischen einem im Endoskopschaft vorgesehenen Systemrohr und der Abdeckung lichtdicht verschließt, um zu verhindern, dass reflektiertes Beleuchtungslicht in das Systemrohr gelangt. Andererseits dient der Ring dazu, dass auch bei starken thermischen Ausdehnungen des Systemrohrs die auf die Abdeckung einwirkenden Kräfte so gering bleiben, dass eine Zerstörung der Abdeckung durch thermische Spannungen nicht zu befürchten ist.

Ausgehend hiervon ist es Aufgabe der Erfindung, ein Endoskop der eingangs genannten Art so weiter zu bilden, daß dauerhaft gute Abbildungseigenschaften gewährleistet werden können.

Erfindungsgemäß wird die Aufgabe bei einem Endoskop der eingangs genannten Art dadurch gelöst, daß mittels eines elastisch ausgebildeten Dichtringes der Bereich zwischen der Abdeckung und dem am nähesten an der Abdeckung liegenden Optikelement der Abbildungsoptik gegenüber dem restlichen Innenraum des Endoskopschaftes abgedichtet ist, wobei der Dichtring am Umfang des am nähesten an der Abdeckung liegenden optischen Elementes anliegt.

Mit einem solchen Dichtring kann sicher vermieden werden, daß in den Zwischenraum Verschmutzungen eindringen können.

Ferner kann so ein sehr kompakter Aufbau verwirklicht werden, bei dem sicher die Verschmutzung des Zwischenraums vermieden werden kann. Des weiteren kann der Dichtring noch als Streulichtschutz für das am nähesten an der Abdeckung liegenden Optikelementes dienen, da er am Umfang austretendes Licht abschattet.

Der Dichtring kann an der der Abbildungsoptik zugewandten Innenseite der Abdeckung anliegen.

Der Dichtring ist elastisch ausgebildet. Er kann aus einem elastischen Polymer hergestellt sein, wie z.B. Ethylen-Propylen-Dien-Kautschuk, Silikon, Teflon, Fluorelastomere, Fluorkautschuk.

Der Dichtring kann als dämpfendes Element zwischen Abdeckung und Abbildungsoptik dienen, daß z.B. bei Stößen gegen die Abdeckung die Abbildungsoptik aufgrund seiner dämpfenden Eigenschaft schützt.

Der Dichtring kann kreisringförmig, oval, oder eine sonstige in sich geschlossene Form aufweisen. Wesentlich ist, daß er den Zwischenraum sicher abdichtet.

Die Abbildungsoptik kann in axialer Richtung gegenüber der Abdeckung verschiebbar sein. Damit können unterschiedliche Ausdehnungen während des Autoklavierens berücksichtigt werden. In diesem Fall dient der Dichtring als Toleranzausgleich, so daß selbst während dieser unterschiedlichen Längenänderungen der Zwischenraum immer sicher abgedichtet ist.

Der Endoskopschaft kann ein Außenrohr und ein im Außenrohr angeordnetes Innenrohr aufweisen, an dessen distalen Ende die Beobachtungsöffnung ausgebildet ist. Insbesondere kann die Abbildungsoptik in einem Optikrohr sitzen, das im Innenrohr angeordnet ist.

Der Dichtring kann als O-Ring oder als Ring mit ovalem oder rundem Querschnitt ausgebildet sein. Es ist jedoch auch jeder andere Ringquerschnitt möglich, wie z.B. quadratisch, rechteckig, polygonförmig, ...

Bei dem erfindungsgemäßen Endoskop kann am distalen Ende ein Träger angeordnet sein, der eine Mittelöffnung aufweist, die die Beobachtungsöffnung bildet. Die Mittelöffnung ist mittels der transparenten Abdeckung hermetisch dicht verschlossen. Der Träger ist bevorzugt nicht transparent und seinerseits hermetisch dicht mit dem Endoskopschaft oder z.B. dem Innenrohr verbunden. Der Dichtring kann am Träger statt an der Abdeckung anliegen.

Das Endoskop kann als Endoskop mit starrem oder biegbarem Endoskopschaft ausgebildet sein. Insbesondere kann das Endoskop ein medizinisches Endoskop sein. Ferner kann ein Bildsensor unmittelbar hinter der Abbildungsoptik angeordnet sein, so daß ein sehr kompaktes Bildaufnahmemodul am distalen Ende des Endoskopschaftes realisiert werden kann.

Das erfindungsgemäße Endoskop kann weitere, dem Fachmann bekannte Elemente aufweisen, die zum bestimmungsgemäßen Gebrauch des Endoskops notwendig sind.

Es versteht sich, daß die vorstehend genannten und die nachstehend noch zu erläuternden Merkmale nicht nur in den angegebenen Kombinationen, sondern auch in anderen Kombinationen oder in Alleinstellung einsetzbar sind, ohne den Rahmen der vorliegenden Erfindung zu verlassen.

Nachfolgend wird die Erfindung beispielsweise anhand der beigefügten Zeichnungen, die auch erfindungswesentliche Merkmale offenbaren, noch näher erläutert. Es zeigen:
- Fig. 1: eine schematische Darstellung einer Ausführungsform des erfindungsgemäßen Endoskopes, und
- Fig. 2: eine vergrößerte Schnittdarstellung des Details A von Fig. 1.

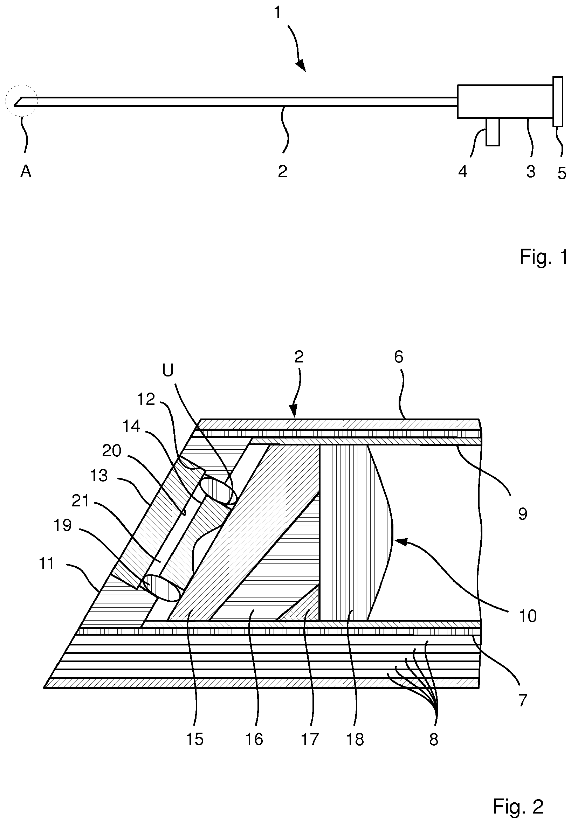

Bei der in Fig. 1 und 2 gezeigten Ausführungsform umfaßt das erfindungsgemäße Endoskop 1 einen Endoskopschaft 2, der mit seinem proximalen Ende mit einem Hauptkörper 3 verbunden ist. Der Hauptkörper 3 weist einen Lichtleiteranschluß 4 sowie einen Adapter 5 zur Verbindung einer Kamera (nicht gezeigt) mit dem Hauptkörper 3 auf.

Wie insbesondere der vergrößerten Schnittdarstellung des distalen Endes des Endoskopschaftes 2 in Fig. 2 zu entnehmen ist, weist der Endoskopschaft 2 ein Außenrohr 6 auf, in dem ein Innenrohr 7 mit geringerem Durchmesser eingesetzt ist. In dem Bereich zwischen Außen- und Innenrohr 6, 7 sind schematisch eingezeichnete Lichtleiter 8 angeordnet.

Innerhalb des Innenrohrs 7 ist ein Optikrohr 9 angeordnet, das eine Abbildungsoptik 10 an seinem distalen Ende trägt. Das distale Ende des Optikrohrs 9 ist zusammen mit der Abbildungsoptik 10 etwas gegenüber dem distalen Ende von Innen- und Außenrohr 7, 8 zurückgesetzt. Im Bereich des distalen Endes des Innenrohrs 7 ist ein nicht transparenter Träger 11 hermetisch dicht (= dicht beim Autoklavieren) mit der Innenseite des Innenrohrs 7 verbunden. Der Träger 11 weist eine Mittelöffnung 12 auf, die im Querschnitt gesehen, gestuft ausgebildet ist. In die Mittelöffnung 12 ist eine transparente Abdeckung 13 (hier in Form eines Saphirglases) eingesetzt und mit dem Träger 11 verlötet, so daß die Mittelöffnung 12 und damit das distale Ende des Innenrohrs 7 hermetisch dicht verschlossen ist. Die Mittelöffnung 12 dient als Beobachtungsöffnung für die Abbildungsoptik 10.

Die Abbildungsoptik 10 weist, wie aus Fig. 2 ersichtlich ist, fünf Optikelemente 14, 15, 16, 17 und 18 auf, von denen die Optikelemente 15 - 18 mit der Innenseite des Optikrohrs 9 verbunden sind. Da das Optikrohr 9 einen kreisförmigen Querschnitt aufweist und die Optikelemente 15 - 17 keinen kreisförmigen Querschnitt aufweisen, ist in einer Richtung senkrecht zur Zeichenebene von Fig. 2 zwischen den Optikelementen 15 - 18 und dem Optikrohr 9 ein Zwischenraum vorhanden. Um unerwünschtes Streulicht zu vermeiden, das seitlich aus den Optikelemente 14 - 18 austreten kann, sind die Ränder der Optikelemente 14 - 18 geschwärzt. Das erste Optikelement 14, das am nähesten zu der Abdeckung 13 angeordnet und hier eine Negativ- bzw. Zerstreuungslinse ist, ist mit dem zweiten Optikelement 15 verklebt.

Um das erste Optikelement 14 ist ein ringförmiger Dichtring bzw. Dichtungsring 19 angeordnet, der einerseits am Umfang bzw. Rand U des ersten Optikelementes 14 und andererseits an der Innenseite 20 der Abdeckung 13 anliegt. Dadurch wird der Zwischenraum 21 zwischen der Innenseite 20 und dem ersten Optikelement 14 gegenüber dem restlichen Innenraum des Optikrohrs 9 abgedichtet. Dadurch kann vorteilhaft vermieden werden, daß Verschmutzungen von der proximalen Seite (also von rechts in Fig. 2) aus dem Innenrohr 7 oder dem Optikrohr 9 in den Zwischenraum 21 gelangen, was bei der optischen Abbildung sehr störend wäre. Insbesondere können Abblätterungen der Schwärzungen der Ränder der Optikelemente 14 - 18 nicht in den Zwischenraum 21 gelangen.

Ferner dient der Dichtring 19 zur Streulichtunterdrückung bei dem ersten Optikelement 14 und dient als Toleranzausgleich beim Autoklavieren, wenn sich das Optikrohr 9 mit der Abbildungsoptik 10 anders in axialer Richtung ausdehnt als Außen- und Innenrohr 6, 7 zusammen mit dem Träger 11 und der Abdeckung 13.

Der Dichtring 19 ist aus einem elastischen Polymer hergestellt, das so gewählt ist, daß es für die Temperaturen, die beim Autoklavieren auftreten, beständig ist.

Mit dem erfindungsgemäßen Endoskop 1 kann ein vor dem distalen Ende angeordnetes Objekt durch die Abdeckung 13 mittels der Abbildungsoptik 10, die auch als Kompaktobjektiv bezeichnet werden kann, auf einen Bildsensor abgebildet werden, der entweder direkt hinter der Abbildungsoptik 10 (in Fig. 2 also rechts davon) oder am Adapter 5 angeordnet ist. Im letzteren Fall können zwischen der Abbildungsoptik 10 und dem Hauptkörper 3 innerhalb des Endoskopschaftes 2 noch weitere Optiken, wie z.B. bekannte Stablinsen, angeordnet sein.

## Patentansprüche

1. Endoskop mit einem Endoskopschaft (2),
der an seinem distalen Ende eine Beobachtungsöffnung (12) aufweist, die mit einer transparenten Abdeckung (13) hermetisch dicht verschlossen ist,
und mit einer mehrere Optikelemente (14, 15, 16, 17, 18) umfassenden Abbildungsoptik (10), die innerhalb des Endoskopschaftes (2) im Bereich seines distalen Endes von der Abdeckung (13) beabstandet angeordnet ist, und mit einem Dichtring (19), der einen Bereich (21) zwischen der Abdeckung (13) und dem am nähesten an der Abdeckung liegenden Optikelement (14) der Abbildungsoptik (10) gegenüber dem restlichen Innenraum des Endoskopschaftes (2) abdichtet,
wobei der Dichtring am Umfang des am nähesten an der Abdeckung (13) liegenden Optikelementes (14) anliegt, **dadurch gekennzeichnet, dass** der Dichtring (19) elastisch ausgebildet ist.

2. Endoskop nach Anspruch 1, **dadurch gekennzeichnet, daß** der Dichtring an der der Abbildungsoptik (10) zugewandten Innenseite (20) der Abdeckung (13) anliegt.

3. Endoskop nach einem der obigen Ansprüche, **dadurch gekennzeichnet, daß** die Abbildungsoptik (10) in axialer Richtung gegenüber der Abdeckung (13) verschiebbar ist.

4. Endoskop nach einem der obigen Ansprüche, **dadurch gekennzeichnet, daß** der Endoskopschaft (2) ein Außenrohr (6) und ein im Außenrohr (6) angeordnetes Innenrohr (7) aufweist, dessen distales Ende die Beobachtungsöffnung (12) bildet.

5. Endoskop nach Anspruch 4, **dadurch gekennzeichnet, daß** die Abbildungsoptik (10) in einem Optikrohr (9) sitzt, das im Innenrohr (7) angeordnet ist.

6. Endoskop nach einem der obigen Ansprüche, **dadurch gekennzeichnet, daß** der Dichtring (19) als O-Ring ausgebildet ist.

## Claims

1. Endoscope having an endoscope shaft (2),
which has an observation opening (12) on its distal end, which is closed hermetically sealed using a transparent cover (13),
and having an imaging optic (10), comprising multiple optic elements (14, 15, 16, 17, 18), which is situated inside the endoscope shaft (2) in the area of its distal end spaced apart from the cover (13),
and having a sealing ring (19), which seals an area (21) between the cover (13) and the optic element (14) of the imaging optic (10) closest to the cover in relation to the remaining inner chamber of the endoscope shaft (2),
wherein the sealing ring presses against the circumfence of the optic element (14) closest to the cover (13), **characterized in that** the sealing ring (19) is implemented as elastic.

2. Endoscope according to claim 1, **characterized in that** the sealing ring presses against the inner side (20) of the cover (13) facing toward the imaging optic (10).

3. Endoscope according to any of the above claims, **characterized in that** the imaging optic (10) is displaceable in relation to the cover (13) in the axial direction.

4. Endoscope according to any of the above claims, **characterized in that** the endoscope shaft (2) has an outer tube (6), and an inner tube (7), which is situated in the outer tube (6) and whose distal end forms the observation opening (12).

5. Endoscope according to claim 4, **characterized in that** the imaging optic (10) is seated in an optic tube (9), which is situated in the inner tube (7).

6. Endoscope according to any of the above claims, **characterized in that** the sealing ring (19) is implemented as an O-ring.

## Revendications

1. Endoscope comprenant une tige d'endoscope (2) qui présente à son extrémité distale une ouverture d'observation (12) qui est fermée de manière hermétiquement étanche avec un recouvrement transparent (13), et comprenant une optique d'imagerie (10) comprenant plusieurs éléments optiques (14, 15, 16, 17, 18) qui sont disposés à l'intérieur de la tige d'endoscope (2) dans la région de son extrémité distale à distance du recouvrement (13), et comprenant une bague d'étanchéité (19) qui étanchéifie une région (21) entre le recouvrement (13) et l'élément optique (14) de l'optique d'imagerie (10) le plus proche du recouvrement par rapport au reste de l'espace interne de la tige d'endoscope (2), la bague d'étanchéité s'appliquant contre la périphérie de l'élément optique (14) le plus proche du recouvrement (13), **caractérisé en ce que** la bague d'étanchéité (19) est réalisée sous forme élastique.

2. Endoscope selon la revendication 1, **caractérisé en ce que** la bague d'étanchéité s'applique contre le côté intérieur (20) du recouvrement (13) tourné vers l'optique d'imagerie (10).

3. Endoscope selon l'une quelconque des revendications précédentes, **caractérisé en ce que** l'optique d'imagerie (10) peut être déplacée dans la direction axiale par rapport au recouvrement (13) .

4. Endoscope selon l'une quelconque des revendications précédentes, **caractérisé en ce que** la tige d'endoscope (2) présente un tube extérieur (6) et un tube intérieur (7) disposé dans le tube extérieur (6), dont l'extrémité distale forme l'ouverture d'observation (12).

5. Endoscope selon la revendication 4, **caractérisé en ce que** l'optique d'imagerie (10) repose dans un tube d'optique (9) qui est disposé dans le tube intérieur (7).

6. Endoscope selon l'une quelconque des revendications précédentes, **caractérisé en ce que** la bague d'étanchéité (19) est réalisée sous forme de joint torique.
